(19) Europäisches Patentamt
European Patent Office
Office européen des brevets

(11) Veröffentlichungsnummer: **0 033 066**
**A2**

(12) # EUROPÄISCHE PATENTANMELDUNG

(21) Anmeldenummer: **81100049.6**

(22) Anmeldetag: **07.01.81**

(51) Int. Cl.³: **C 07 D 499/68**
C 07 D 307/20, C 07 D 307/32
C 07 D 333/32, C 07 D 333/38
C 07 D 309/30, C 07 D 309/10
C 07 D 335/02, A 61 K 31/43

(30) Priorität: **07.01.80 US 110275**

(43) Veröffentlichungstag der Anmeldung:
**05.08.81 Patentblatt 81/31**

(84) Benannte Vertragsstaaten:
**AT BE CH DE FR GB IT LI LU NL SE**

(71) Anmelder: **F. HOFFMANN-LA ROCHE & CO.**
**Aktiengesellschaft**

**CH-4002 Basel(CH)**

(72) Erfinder: **Burri, Kaspar F., Dr.**
**Liebrütistrasse 20**
**CH-4303 Kaiseraugst(CH)**

(72) Erfinder: **Rosen, Perry**
**Sunset Drive 26**
**North Caldwell, N.J.(US)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80(DE)**

(54) **Penicillinderivate, Zwischenprodukte für ihre Herstellung, pharmazeutische Präparate enthaltend dieselben, ihre Herstellung, sowie ihre Verwendung.**

(57) Die vorliegende Erfindung betrifft Acylderivate der Formel

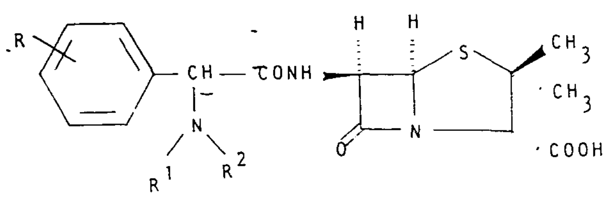

II

worin R Wasserstoff oder Hydroxy und R¹ und R² unabhängig voneinander Wasserstoff oder einen unsubstituierten oder einfach oder mehrfach durch niederes Alkyl und/oder Halogen substituierten, gesättigten, ein Heteroatom enthaltenden, heterocyclischen 5- oder 6-gliedrigen Ring bedeuten, der als Heteroatom ein Sauerstoff- oder Schwefelatom enthält, wobei der besagte Ring über das dem besagten Heteroatom benachbarte Kohlenstoffatom mit dem Stickstoffatom verknüpft ist, mit der Massgabe, dass mindestens einer der Reste R¹ und R² nicht Wasserstoff bedeutet, sowie leicht hydrolysierbare Ester und Salze davon und Hydrate von Verbindungen der Formel II und ihren Estern und Salzen.

Die vorliegende Erfindung umfasst auch ein Verfahren zur Herstellung obiger Acylderivate und pharmazeutische Präparate enthaltend solche Verbindungen. Darüberhinaus betrifft die Erfindung die Verwendung obiger Acylderivate bei der Bekämpfung bzw. Verhütung von Krankheiten, insbesondere Infektionen. Weiter betrifft die vorliegende Erfindung neue, im oben erwähnten Verfahren nützliche Ausgangsstoffe.

EP 0 033 066 A2

F.Hoffmann-La Roche & Co.Aktiengesellschaft,Basel,Schweiz



RAN 4410/140

# Penicillinderivate, Zwischenprodukte für ihre Herstellung, pharmazeutische Präparate enthaltend dieselben, ihre Herstellung, sowie ihre Verwendung.

6-[D(-)-α-Amino-phenylacetamido]-penicillansäuren der Formel

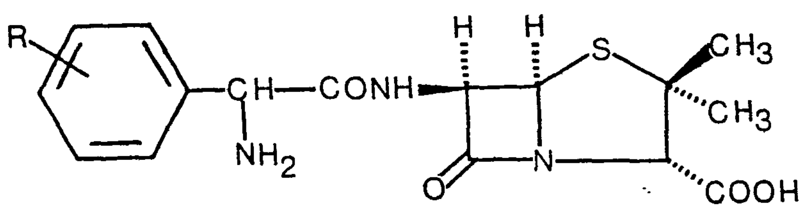

I

worin R Wasserstoff oder Hydroxy bedeutet, besitzen ein weites Wirkungsspektrum gegen gram-positive und gram-negative Bakterien. Die orale Verabreichung führt zu hohen Blutspiegeln. Unter den Verbindungen die von der Formel I umfasst werden sind Amoxicillin und Ampicillin.

Die einzigen erhältlichen Dosierungsformen von Verbindungen der Formel I sind jedoch für die parenterale oder intravenöse Verabreichung mittels wasserlöslicher Salze. Diese Salze haben in wässrigen Lösungen nur eine sehr kurze Halbwertszeit.

Mn /17.11.80

Kondensationsprodukte von α-Aminopenicillinen mit verschiedenen Aldehyden und Ketonen sind bekannt. Derivate von verschiedenen α-Aminopenicillinen mit Nitro-substituierten heterocyclischen Aldehyden sind beispielsweise in US-Patent Nr. 3,657,781 offenbart. Weitere Patente und Patent-Publikationen, welche Derivate von α-Aminopenicillinen mit Aldehyden und Ketonen offenbaren, sind US-Patente Nr. 3,198,804 und 3,558,602 (verschiedene Aldehyde und Ketone), US-Patent Nr. 3,198,788 (Formaldehyd), US-Patent Nr. 3,230,214 (aromatische oder heteroaromatische Aldehyde, welche einen o-Hydroxyl-Substituenten enthalten), US-Patent Nr. 3,325,479 (Diketone), US-Patente Nr. 3,489,746, 3,549,746, 3,814,800 und U.K.-Patent Nr. 1,224,619 (Aceton), US-Patent Nr. 3,725,389 (N-substituierte 4-Piperidone), US-Patent Nr. 3,888,848 (Chloralhydrat), DOS 2,258,994 (unter verschiedenen Aldehyden auch Acetaldehyd) und südafrikanisches Patent Nr. 72/8474 (Acetaldehyd).

Diese Kondensationen von α-Aminopenicillinen der Formel I mit Aldehyden oder Ketonen führt zur Bildung von Imidazolidinyl-Verbindungen, schiffschen Basen oder Mischungen davon. Diese Kondensationsprodukte sind in Wasser leicht löslich und stabiler als die entsprechenden Salze. Sie sind eigentlich biologische Vorläufer der entsprechenden α-Aminopenicilline, da sie in vivo leicht in die entsprechenden α-Aminopenicilline übergeführt werden. Die in vivo Freisetzung der Ketone und Aldehyde kann jedoch nachteilige und/oder toxische Nebenwirkungen verursachen.

Es ist deshalb eine Aufgabe der vorliegenden Erfindung, neue wasserlösliche Formen von α-Aminopenicillinen, welche in wässrigen Systemen stabil sind und keine im Falle der Aldehyde und Ketone schädlichen Nebenwirkungen entfalten, für die orale und parenterale Verabreichung bereitzustellen.

Die vorliegende Erfindung betrifft Penicillinderivate, welche in wässrigen Lösungen eine erhöhte Stabilität aufweisen und welche als antibakterielle therapeutische Mittel nützlich sind. Diese Derivate besitzen die folgende allgemeine Formel:

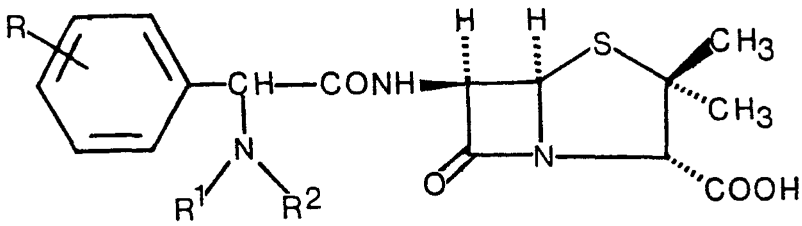

II

worin R Wasserstoff oder Hydroxy und $R^1$ und $R^2$ unabhängig voneinander Wasserstoff oder einen unsubstituierten oder einfach oder mehrfach durch niederes Alkyl und/oder Halogen substituierten, gesättigten, ein Heteroatom enthaltenden, heterocyclischen 5- oder 6-gliedrigen Ring bedeuten, der als Heteroatom ein Sauerstoff- oder Schwefelatom enthält, wobei der besagte Ring über das dem besagten Heteroatom benachbarte Kohlenstoffatom mit dem Stickstoffatom verknüpft ist, mit der Massgabe, dass mindestens einer der Reste $R^1$ und $R^2$ nicht Wasserstoff bedeutet.

Ebenfalls Gegenstand der vorliegenden Erfindung sind leicht hydrolysierbare Ester und Salze von Verbindungen der allgemeinen Formel II und Hydrate von Verbindungen der allgemeinen Formel II und ihren Estern und ihren Salzen.

Der in der vorliegenden Beschreibung verwendete Ausdruck "niederes Alkyl" bezeichnet gesättigte aliphatische Kohlenwasserstoffradikale mit 1-7 Kohlenstoffatome, wie Methyl, Aethyl, n-Propyl, Isopropyl, Butyl, usw. Der Ausdruck "Halogen" bedeutet die vier Formen Chlor, Fluor, Jod und Brom, wobei Chlor und Fluor besonders bevorzugt sind.

Die neuen und nützlichen erfindungsgemässen Produkte umfassen Verbindungen der allgemeinen Formel II, ihre

Salze, Hydrate und Ester davon. Jedes herkömmliche, pharmazeutisch annehmbare Salz oder jeder herkömmliche, pharmazeutisch annehmbare Ester wird von Verbindungen der allgemeinen Formel II umfasst.

Die Verbindungen der vorliegenden Erfindung umfassen die pharmazeutisch annehmbaren nicht toxischen Ester von Verbindungen der allgemeinen Formel II. Geeignete Ester sind solche, welche im Körper leicht zur ursprünglichen Säure hydrolysiert werden, beispielsweise niedere Alkoxyniedere Alkylester, wie Methoxymethylester, Acyloxy-niedere Alkylester, wie Acetoxymethyl-, Pivaloyloxyäthyl-, α-Acetoxyäthyl-, α-Acetoxybenzyl- und α-Pivaloyloxyäthyl-Ester; Benzylester; niedere Alkoxycarbonyloxy-niedere Alkylester, wie Aethoxycarbonyloxymethyl- und α-Aethoxycarbonyloxyäthyl-Ester; und Lacton-, Thiolacton- und Dithiolacton-Ester. Niedere Alkanoyloxyester sind ebenfalls geeignet.

Geeignete Salze von Verbindungen der allgemeinen Formel II sind Metallsalze, beispielsweise Alkalimetallsalze, wie Natrium- oder Kaliumsalze, Erdalkalimetallsalze, wie Calcium- oder Magnesiumsalze, Ammonium- oder substituierte Ammoniumsalze, beispielsweise mit niederen Alkylaminen, wie Triäthylamin, Hydroxyniederalkylaminen, wie 2-Hydroxyäthylamin, bis-(2-Hydroxyäthyl)-amin, tris-(Hydroxymethyl)amin oder tris-(2-Hydroxyäthyl)-amin, Cycloalkylaminen, wie Bicyclohexylamin, Procain, Dibenzylamin, N,N-Dibenzyläthylendiamin, 1-Ephenamin, N-Aethylpiperidin, N-Benzyl-beta-phenäthylamin, Dehydroabiethylamin, N,N'-bis-Dehydroabiethyläthylendiamin, oder mit Basen vom Pyridintyp, wie Pyridin, Collidin oder Chinolin, oder mit anderen Aminen, welche für die Herstellung von Salzen mit Benzylpenicillin verwendet worden sind.

Einer der Reste $R^1$ oder $R^2$ oder beide Reste sind heterocyclische Ringe; diese heterocyclischen Ringe besitzen beispielsweise die Formel:

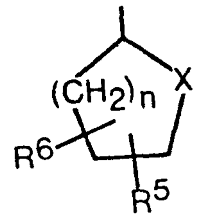

worin n die Zahl 1 oder 2, X Schwefel oder Sauerstoff, $R^5$ Wasserstoff, Halogen oder niederes Alkyl und $R^6$ Wasserstoff, Halogen oder niederes Alkyl bedeuten. Die bevorzugten heterocyclischen Ringe sind Tetrahydrofuran-2-yl, 2H-Tetrahydropyran-2-yl, Tetrahydrothiophen-2-yl und 2H-Tetrahydro-thiopyran-2-yl. Besonders bevorzugte heterocyclische Ringe sind die obigen Gruppen, welche unsubstituiert, nur mit Halogen substituiert, nur durch niederes Alkyl substituiert und durch Halogen und niederes Alkyl substituiert sind.

Die Verbindungen der allgemeinen Formel II und die entsprechenden Ester, Salze und Hydrate können erfindungsgemäss dadurch hergestellt werden, dass man

a) zur Herstellung einer Verbindung der allgemeinen Formel II, ein Alkalimetallsalz einer Verbindung der allgemeinen Formel

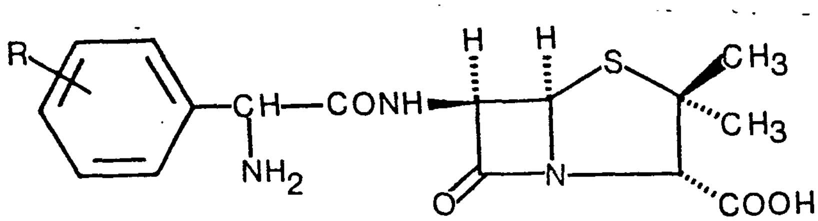

I

worin R Wasserstoff oder Hydroxy bedeutet, mit einer Verbindung der Formel

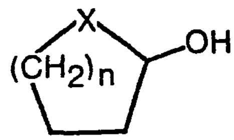

III

worin X Sauerstoff oder Schwefel und n die Zahl 1 oder 2 bedeuten, und die in einer oder in mehreren Stellungen durch niederes Alkyl und/oder Halogen substituiert sein kann, umsetzt,

oder

b) zur Herstellung eines leicht hydrolysierbaren Esters einer Verbindung der allgemeinen Formel II, eine Carbonsäure der allgemeinen Formel II entsprechend verestert,

oder

c) zur Herstellung von Salzen oder Hydraten von Verbindungen der allgemeinen Formel II, oder zur Herstellung von Hydraten dieser Salze, eine Verbindung der allgemeinen Formel II in ein Salz oder in ein Hydrat oder in ein Hydrat eines solchen Salzes überführt.

Eine Verbindung der Formel I reagiert mit einer Verbindung der Formel III zu einer Verbindung der Formel II. Im allgemeinen wird diese Reaktion vorzugsweise in einem wässrigen Medium durchgeführt. In der Regel wird diese Reaktion in der Gegenwart einer anorganischen Alkalimetallbase, wie Natriumhydroxid, Kaliumhydroxid, Lithiumhydroxid, und dergleichen durchgeführt. Temperatur und Druck sind für diese Reaktion nicht kritisch. Man kann die Reaktion bei Raumtemperatur und Atmosphärendruck durchführen. Erwünschtenfalls können auch höhere oder niedrigere Temperaturen verwendet werden, beispielsweise Temperaturen in einem Bereich von etwa 0°C bis etwa 50°C.

Nach Durchführung der Reaktion zwischen Verbindungen

der Formel I und III kann die Verbindung der Formel II in Form der freien Säure aus dem Reaktionsgemisch isoliert werden, indem man das Reaktionsgemisch vor der Isolierung der Verbindung der Formel II neutralisiert. Andererseits erhält man das Salz einer Verbindung der Formel II, wenn das Reaktionsprodukt ohne vorherige Neutralisation isoliert wird.

Um einen leicht hydrolysierbaren Ester einer Carbonsäure der Formel II, gemäss Variante b) des erfindungsgemässen Verfahrens, herzustellen, wird eine Carbonsäure der Formel II vorzugsweise mit einem entsprechenden Halogenid, vorzugsweise einem Iodid, das die gewünschte Estergruppe enthält, umgesetzt. Die Reaktion kann mit Hilfe einer Base, wie einem Alkalimetallhydroxid, einem Alkalimetallcarbonat oder einem organischen Amin (z.B. Triäthylamin), beschleunigt werden. Die Veresterung wird vorzugsweise in einem inerten organischen Lösungsmittel, wie Aceton, Dimethylacetamid, Hexamethylphosphorsäuretriamid, Dimethylsulfoxid, oder, insbesondere, Dimethylformamid, durchgeführt. Die Reaktion wird vorzugsweise in einem Temperaturbereich von etwa 0°C bis etwa 40°C durchgeführt.

Die Herstellung von Salzen und Hydraten von Verbindungen der Formel II oder von Hydraten der besagten Salze, gemäss Variante c) des erfindungsgemässen Verfahrens, kann in an sich bekannter Weise erfolgen; beispielsweise durch Umsetzen einer Carbonsäure der Formel II mit einer äquivalenten Menge einer gewünschten Base, zweckmässigerweise in einem Lösungsmittel, wie Wasser, oder in einem organischen Lösungsmittel (z.B. Aethanol, Methanol, Aceton oder dergleichen). Die Temperatur ist bei der Herstellung der Salze nicht kritisch. Die Salzbildung wird in der Regel bei Raumtemperatur durchgeführt, aber sie kann auch bei Temperaturen die leicht darüber oder darunter liegen durchgeführt werden, beispielsweise in einem Bereich von etwa 0°C bis etwa 50°C.

Die Herstellung der Hydrate erfolgt üblicherweise automatisch im Verlauf der Herstellungsverfahren, oder erfolgt als Folge der hygroskopischen Eigenschaften eines ursprünglich wasserfreien Produktes. Zur kontrollierten Herstellung eines Hydrates wird eine ganz oder teilweise wasserfreie Carbonsäure der Formel II oder ein Ester oder ein Salz davon einer feuchten Atmosphäre ausgesetzt, z.B. bei etwa +10°C bis 40°C.

Die Verbindungen der Formel III sind teilweise bekannte Verbindungen. Im allgemeinen können die Niederalkyl- und halogensubstituierten Ringverbindungen der Formel III, worin der Halogensubstituent sich in α-Stellung zur Hydroxygruppe befindet, d.h. eine Verbindung der Formel:

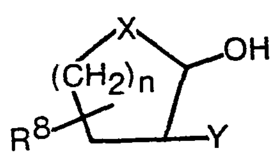

IV

worin $R^8$ niederes Alkyl, X Schwefel oder Sauerstoff, Y Halogen und n die Zahl 1 oder 2 bedeuten,

aus Verbindungen der Formel:

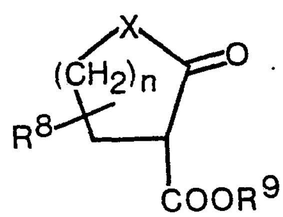

V

worin $R^8$ und n obige Bedeutung besitzen und $R^9$ niederes Alkyl bedeutet,

über die folgenden Zwischenprodukte:

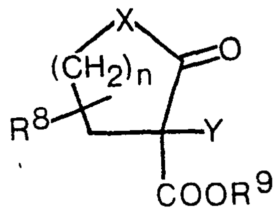

VI

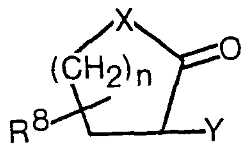

VII

worin $R^8$, $R^9$, X, Y und n obige Bedeutung besitzen, hergestellt werden.

Die Verbindung der Formel V wird in eine Verbindung der Formel VI übergeführt, indem man die Verbindung der Formel V mit einem Halogenierungsmittel behandelt. Jedes herkömmliche Halogenierungsmittel kann für diese Reaktion verwendet werden. Unter den bevorzugten Halogenierungsmitteln ist Perchlorylfluorid, das ein Fluorierungsmittel ist. Sämtliche Reaktionsbedingungen welche üblicherweise bei der Verwendung dieser Halogenierungsmittel verwendet werden, können für die Ueberführung einer Verbindung der Formel V in eine solche der Formel VI verwendet werden. Die Verbindung der Formel VI wird durch Hydrolyse und Decarboxylierung in eine Verbindung der Formel VII übergeführt. Die Decarboxylierung kann nach herkömmlichen, bekannten Methoden durchgeführt werden. In der Regel wird die Decarboxylierung durchgeführt, indem man die Verbindung der Formel VI in Form der freien Säure während 5 bis 10 Stunden auf Temperaturen von 120°C bis 160°C erhitzt.

Die Verbindung der Formel VII wird in eine Verbindung der Formel IV übergeführt, indem man die Verbindung der Formel VII mit einem Reduktionsmittel behandelt. Unter den Reduktionsmitteln, welche für diese Reaktion verwendet

werden können, sind Diisobutylaluminiumhydride und Disiamylboran (bis-3-Methyl-2-butylboran). Sämtliche Reaktionsbedingungen, welche üblicherweise bei Verwendung dieser Reduktionsmittel verwendet werden, können für die vorliegende Reaktion angewendet werden.

Es wird angenommen, dass die Verbindungen der Formeln IV, VI und VII neu sind.

Die Verbindungen der vorliegenden Erfindung, nämlich Verbindungen der Formel II und ihre entsprechenden leicht hydrolysierbaren Ester, Salze und Hydrate, besitzen ähnliche antibakterielle Wirkungen wie Amoxicillin und Ampicillin in vitro und in vivo. Diese Verbindungen sind wirksam gegen Mikroorganismen, wie S. pyogenes, E. coli und K. pneumoniae.

Die Verbindungen der vorliegenden Erfindung können in geeigneten Dosierungsformen oral, parenteral oder topisch verabreicht werden; sie können auch in Form ihrer Salze oder Ester verabreicht werden. Die Verbindungen der vorliegenden Erfindung sind stabil in Lösung und sind deshalb besonders vorteilhaft, wenn parenterale Verabreichung angezeigt ist.

Die Verbindungen der vorliegenden Erfindung können zusammen mit herkömmlichen verträglichen organischen oder anorganischen pharmazeutischen Trägermaterialien, beispielsweise Wasser, Gelatine, Gummi arabicum, Lactose, Stärke, Magnesiumstearat, Talk, pflanzliche Oele, Polyalkylenglykole, Vaselin und dergleichen, verabreicht werden. Diese pharmazeutischen Präparate können als Einheitsdosierungsformen oder Lösungen vorliegen. Diese Präparate können auch andere therapeutisch wertvolle Substanzen oder herkömmliche pharmazeutische Hilfsstoffe, wie Konservierungsmittel, Stabilisierungsmittel, Netzmittel, Emulgiermittel, Puffer oder dergleichen enthalten.

Die pharmazeutischen Präparate können in herkömmlichen festen Dosierungsformen, wie Tabletten, Kapseln, Dragées und dergleichen, in halbfesten Formen, wie Salben und Crèmes, in herkömmlichen Dosierungsformen, wie Suppositorien, Trockenampullen und dergleichen, vorliegen.

Dosierungsformen für die parenterale Verwendung werden vorbereitet, indem man ein Pulver oder ein Salz einer aktiven Verbindung vor der Verwendung in einem geeigneten sterilen wässrigen Lösungsmittel auflöst. Da die Verbindungen der vorliegenden Erfindung relativ stabil sind, kann eine Lösung 4 Stunden vor der Weiterverwendung hergestellt werden, anstatt unmittelbar vor der Verwendung.

Typische geeignete Lösungsmittel umfassen Wasser für die Injektion. Typische für die intravenöse Verabreichung geeignete Lösungen umfassen Kochsalzinjektionslösungen, Dextroseinjektionslösungen, z.B. 5%ig oder 10%ig, und dergleichen. Die Injektionslösungen können Konservierungsmittel enthalten.

Die Menge an aktiver Verbindung welche für die Behandlung bakterieller Infektionen verwendet wird ist abhängig von den Befürfnissen des Patienten und von der Beurteilung des Klinikers. In der Regel dürfte jedoch die Verabreichung einer Verbindung gemäss vorliegender Erfindung entsprechend den Dosierungsvorschriften für Amoxicillin oder Ampicillin genügend sein, d.h., dem normalen Erwachsenen werden 3-4 Mal täglich etwa 250 mg bis etwa 500 mg verabreicht.

Für die Injektion werden entsprechende Aequivalente von etwa 200 mg bis etwa 500 mg Amoxicillin oder Ampicillin in 1-2 ml 3-4 Mal täglich injiziert.

Orale Dosierungsformen, z.B. Kapseln enthalten entsprechende Aequivalente von etwa 250 mg bis etwa 500 mg Amoxicillin oder Ampicillin. Für die parenterale Verwendung

dürften Konzentrationen von etwa 100 mg pro ml bis etwa 250 mg pro ml annehmbar sein.

Die Verbindungen gemäss vorliegender Erfindung einerseits und Amoxicillin andererseits wurden auf ihre akute Toxizität in Mäusen hin getestet, und zwar bei oraler, subcutaner und intraperitonealer Verabreichung. Die folgenden erfindungsgemässen Verbindungen wurden getestet:

Verbindung A: /2S-[2α,5α,6β(S*)]/-6-/[2-Tetrahydrofuranylamino-(4-hydroxyphenyl)acetyl]amino/-3,3-dimethyl-7-oxo-4-thia-1-azabicyclo[3.2.0]-heptan-2-carbonsäure-Natriumsalz;

Verbindung B: /2S-[2α,5α,6β(S*)]/-6-[bis(Tetrahydro-2H-pyran-2-yl)amino]-/[(4-hydroxyphenyl)acetyl]amino/-3,3-dimethyl-7-oxo-4-thia-1-azabicyclo[3.2.0]heptan-2-carbonsäure-Natriumsalz;

Verbindung C: /2S-[2α,5α,6β(S*)]/-6-/[Tetrahydro-2-thienylamino-(4-hydroxyphenyl)acetyl]amino/-3,3-dimethyl-7-oxo-4-thia-1-azabicyclo[3.2.0]-heptan-2-carbonsäure-Natriumsalz und

Verbindung D: /2S-[2α,5α,6β(S*)]/-6-/bis[5-Aethyl-3-fluor-3-tetrahydrofuranylamino-(4-hydroxyphenyl)acetyl]amino/-3,3-dimethyl-7-oxo-4-thia-1-azabicyclo[3.2.0]heptan-2-carbonsäure-Natriumsalz.

Toxizitätsangaben sind in der untenstehenden Tabelle 1 zusammengestellt.

Tabelle 1

Akute Toxizität in Mäusen

| Verbindung | $LD_{50}$ = (mg/kg) | | |
|---|---|---|---|
| | p.o. | s.c. | i.p. |
| A | 9100 | 4550 | 3217 |
| Amoxicillin | >10000 | >10000 | 3142 |
| B | >6311 | 4462 | 6311 |
| Amoxicillin | >10000 | 7070 | 3535 |
| C | 558 | 598 | 668 |
| Amoxicillin | >10000 | 7070 | 3150 |
| D | 3959 | 2800 | 3519 |
| Amoxicillin | >10000 | 5864 | 3150 |

> = mehr als
p.o. = per os
s.c. = subcutan
i.p. = intraperitoneal

Die Verbindung B, /2S-[2α,5α,6β(S*)]/-6-[bis(Tetrahydro-2H-pyran-2-yl)amino]/[(4-hydroxyphenyl)acetyl]amino/-3,3-dimethyl-7-oxo-4-thia-1-azabicyclo[3.2.0]heptan-3-carbonsäure-Natriumsalz, wurde ausgewählt um die relative Stabilität von wässrigen Lösungen der erfindungsgemässen Verbindungen zu illustrieren, und zwar bei Raumtemperatur und bei 5°C. Die Stabilität der Verbindung wurde in 0,9%-iger Kochsalzlösung, 5%iger wässriger Dextroselösung und in Wasser bei Raumtemperatur und/oder bei 5°C untersucht.

Die Resultate sind in der untenstehenden Tabelle 2 zusammengestellt. Die anfänglichen Konzentrationen der Verbindung B in Lösung wurde auf der Basis von Amoxicillin berechnet.

Tabelle 2

| Lösungsmittel | Konzentration mg/ml | Aufbewahrungsbedingungen | Anteil an unverändertem Material in % |
|---|---|---|---|
| 0.9% Kochsalzlösung | 2.30 | RT, 6 Std. | 96 |
| | | RT, 24 Std. | 91 |
| | | RT, 48 Std. | 84 |
| | 2.25 | RT, 6 Std. | 96 |
| | | RT, 6 Std.+5°C, 192 Std. | 98 |
| | 2.20 | 5°C, 24 Std. | 100 |
| | | 5°C, 48 Std. | 100 |
| | 34 | RT, 6 Std. | 97 |
| | | RT, 24 Std. | 85 |
| | | RT, 48 Std. | 76 |
| | 29.1 | RT, 6 Std. | 97 |
| | | RT, 6 Std.+5°C, 192 Std. | 100 |
| | 31 | 5°C, 24 Std. | 100 |
| | | 5°C, 48 Std. | 100 |
| 5% Dextrose in Wasser | 2.10 | RT, 6 Std. | 95 |
| | | RT, 24 Std. | 89 |
| | | RT, 48 Std. | 71 |
| | 2.10 | RT, 6 Std. | 95 |
| | | RT, 6 Std.+5°C, 192 Std. | 73 |
| | 2.20 | 5°C, 24 Std. | 95 |
| | | 5°C, 48 Std. | 91 |
| | 30 | RT, 6 Std. | 96 |
| | | RT, 24 Std. | 92 |
| | | RT, 48 Std. | 83 |
| | 31 | RT, 6 Std. | 100 |
| | | RT, 6 Std.+5°C, 192 Std. | 81 |
| | 33 | 5°C, 24 Std. | 94 |
| | | 5°C, 48 Std. | 84 |
| wässr. Konzentrat | 155 | 5°C, 24 Std. | 100 |
| | | 5°C, 48 Std. | 100 |

In 0,9-proz. Kochsalzlösung behielten die 0,2-proz. und 3-proz. Lösungen 90% ihrer Aktivität nach mindestens 8 Tagen (192 Stunden) bei 5°C. Bei Raumtemperatur behielt die 0,2-proz. Lösung 90% ihrer Aktivität während etwa 24 Stunden, währenddem die 3-proz. Lösung weniger als 24 Stunden lang eine 90-proz. Aktivität aufwies.

Bei Raumtemperatur behielten beide Lösungen etwa 90% ihrer Aktivität während etwa 24 Stunden.

Die Verbindungen gemäss vorliegender Erfindung sowie Amoxicillin wurden getestet um ihre in vitro Aktivität gegen repräsentative systemisch wirkende grampositive und gramnegative Bakterien zu ermitteln. MIC ist die minimale Hemmkonzentration (Minimum Inhibitory Concentration). MBC ist die minimale bakterizid wirksame Konzentration (Minimum Bactericidal Concentration).

In der Tabelle 3a sind die oben definierten MIC-Werte und in der Tabelle 3b die oben definierten MBC-Werte zusammengestellt.

Tabelle 3(a)

In vitro Activität

a) Minimale Hemmkonzentration (MIC) in µg/ml

| Organismus | Amoxicillin | A | Amoxicillin | B | Amoxicillin | C | Amoxicillin | D |
|---|---|---|---|---|---|---|---|---|
| Streptococcus pyogenes | .098 | .022 | 0.31 | 0.39 | 0.195 | 0.554 | 0.024 | 0.027 |
| Streptocpccus pneumoniae I | .012 | .022 | 0.039 | 0.049 | - | - | - | - |
| Staphylococcus aureus | .195 | .177 | 0.078 | 0.098 | 0.195 | 0.138 | 0.098 | 0.055 |
| Escherichia coli | 6.25 | 5.69 | 3.125 | 3.94 | 3.125 | 4.44 | 3.125 | 3.5 |
| Klebsiella pneumoniae | .39 | .71 | 0.39 | 0.09 | 0.39 | 0.554 | 0.195 | 0.218 |
| Salmonella schottmuelleri | .78 | .71 | 0.78 | 0.98 | - | - | - | - |
| Salmonella typhosa | .39 | .35 | 0.31 | 0.12 | - | - | - | - |
| Proteus vulgari | - | - | - | - | 1.56 | 0.554 | - | - |
| Streptococcus pneumoniae II | - | - | - | - | - | - | 0.024 | 0.013 |

Tabelle 3(b)

In vitro Activität

b) Minimale bakterizide Konzentration (MBC) in µg/ml

| Organismus | Amoxicillin | A | Amoxicillin | B | Amoxicillin | C | Amoxicillin | D |
|---|---|---|---|---|---|---|---|---|
| Streptococcus pyogenes | 3.125 | 2.84 | 5.0 | 6.31 | >6.25 | >8.88 | >0.39 | >0.218 |
| Streptococcus pneumoniae I | .024 | .022 | 0.039 | 0.049 | - | - | - | - |
| Staphylococcus aureus | .195 | .177 | 0.078 | 0.098 | 0.195 | 0.138 | 0.098 | 0.055 |
| Escherichia coli | 6.25 | 5.69 | 3.12 | 3.94 | 3.125 | 4.44 | 3.125 | 3.5 |
| Klebsiella pneumoniae | .78 | .71 | 0.39 | 0.98 | 0.39 | 1.108 | 0.195 | 0.437 |
| Salmonella schottmuelleri | .78 | 1.42 | 0.78 | 0.98 | - | - | - | - |
| Salmonella typhosa | .39 | .35 | 0.31 | 0.12 | - | - | - | - |
| Proteus vulgari | - | - | - | - | 0.78 | 0.554 | - | - |
| Streptococcus pneumoniae II | - | - | - | - | - | - | 0.049 | 0.027 |

Die Verbindungen gemäss vorliegender Erfindung, sowie Amoxicillin wurden getestet um ihre in vivo Aktivität gegen repräsentative systemische Infektionen durch grampositive und gramnegative Bakterien in Mäusen zu ermitteln; und zwar durch orale und subcutane Verabreichung. Der $PD_{50}$-Wert ist diejenige Dosis, welche 50% der Tiere vor dem Organismus schützt. Die Resultate sind in der Tabelle 4 zusammengestellt.

Tabelle 4

In vivo Activität

a) Subcutane Verabreichung

$PD_{50}$

| Organismus | Amoxicillin | A | Amoxicillin | B | Amoxicillin | C | Amoxicillin | D |
|---|---|---|---|---|---|---|---|---|
| S. pyogenes | 1.2 | 7.0 | 2.5 | 3.4 | 3.5 | 4.1 | 2.3 | 4.4 |
| S. pneumoniae I | .35 | .7 | 0.6 | 0.7 | 3.0 | 2.5 | 0.9 | 0.9 |
| E. coli | 10 | 8.2 | 5.9 | 18 | 7.3 | 19.4 | 6.8 | 10.4 |
| K. pneumoniae | 3.6 | 3.8 | 5 | 5.7 | 5.0 | 5.3 | 3.7 | 2.4 |
| S. schottmuelleri | 1.5 | 2.4 | 4.7 | 5.7 | - | - | - | - |
| S. typhosa | 2.7 | 7.3 | 0.95 | 1.4 | - | - | - | - |
| S. aureus | - | - | < 0.2 | 0.5 | - | - | < 0.2 | 0.34 |
| S. pneumoniae II | - | - | - | - | 1.6 | 3.2 | 0.6 | 0.5 |
| P. mirabilis | - | - | - | - | < 0.5 | 2.5 | 0.6 | 0.4 |
| P. aeruginosa | - | - | - | - | > 200 | > 142 | - | - |

Tabelle 4 (Fortsetzung)

In vivo Activität

b) Orale Verabreichung

$PD_{50}$

| Organismus | Amoxicillin | A | Amoxicillin | B | Amoxicillin | C | Amoxicillin | D |
|---|---|---|---|---|---|---|---|---|
| S. pyogenes | 1.0 | 3.8 | 1.4 | 1.9 | 6.1 | 6.3 | 8.3 | 6.0 |
| S. pneumoniae I | .85 | 1.5 | 0.8 | 0.7 | 2.3 | 4.7 | 1.3 | 0.7 |
| E. coli | 14.1 | 23.8 | 18.5 | 23 | 19.3 | 18.7 | 13.1 | 22.1 |
| K. pneumoniae | 5.0 | 9.9 | 10.8 | 8.6 | 4.3 | 15.5 | 6.1 | 4.8 |
| S. schottmuelleri | 1.8 | 5.4 | 7.6 | 1.9 | - | - | - | - |
| S. typhosa | 13.3 | 8.0 | 3.7 | 3.6 | - | - | - | - |
| S. aureus | - | - | 0.5 | 0.6 | - | - | 0.6 | 1.2 |
| S. pneumoniae II | - | - | - | - | 1.6 | 3.2 | 0.7 | 0.7 |
| P. mirabilis | - | - | - | - | 1.8 | 3.8 | 1.1 | 1.6 |
| P. aeruginosa | - | - | - | - | > 200 | >142 | - | - |

Die folgenden Beispiele erläutern die Erfindung. In den Beispielen bedeutet der Ausdruck "Dibal-H" di-Isobutylaluminiumhydrid. Beim verwendeten Aether handelt es sich um Diäthyläther.

## Beispiel 1

Eine gerührte Suspension von 6,28 g (0,015 Mol) Amoxicillin-Trihydrat in 150 ml destilliertem Wasser wird in einer Portion mit 15 ml 1N wässriger Natronlauge versetzt. Das Amoxicillin löst sich rasch auf; nach 2 Minuten gibt man 2,25 g (0,025 Mol) Tetrahydro-2-furanol hinzu und rührt während 2 Minuten bei 25°C. Die Lösung wird anschliessend mittels eines Trockeneis/Aceton-Bades rasch eingefroren und dann während 22 Stunden lyophilisiert. Man erhält /2S-[2α,5α,6β(S*)]/-6-/[2-Tetrahydrofuranylamino(4-hydroxyphenyl)acetyl]amino/-3,3-dimethyl-7-oxo-4-thia-1-azabicyclo[3.2.0]heptan-2-carbonsäure-Natriumsalz (6,55 g) als weisses Pulver.

Anal. Berechnet für $C_{20}H_{24}N_3NaO_6S$:
C, 52,51; H, 5,29; N, 9,19; S, 7,01; Na, 5,03
Gefunden: C, 52,82; H, 5,90; N, 8,25; S, 6,09; Na, 4,38.

## Beispiel 2

Eine gerührte Suspension von 6,28 g (0,015 Mol) Amoxicillin-Trihydrat in 125 ml destilliertem Wasser wird in einer Portion mit 15 ml 1N wässriger Natronlauge versetzt. Das Amoxicillin löst sich rasch auf; nach 2 Minuten gibt man 4,59 g (0,045 Mol) Tetrahydro-2H-pyran-2-ol hinzu und rührt während 2 Minuten bei 25°C. Die erhaltene Lösung wird anschliessend rasch eingefroren und während 72 Stunden lyophilisiert. Man erhält 8,43 g /2S-[2α,5α,6β(S*))]/-6-[Bis(tetrahydro-2H-pyran-2-yl)amino]/[(4-hydroxyphenyl)-acetyl]amino/-3,3-dimethyl-7-oxo-4-thia-1-azabicyclo[3.2.0]-heptan-2-carbonsäure-Natriumsalz-Hydrat als weisses Pulver.

Anal. Berechnet für $C_{26}H_{34}N_3NaO_7S \cdot 1,3\ H_2O$:
C, 53,93; H, 6,37; N, 7,26; S, 5,54
Gefunden: C, 53,20; H, 6,37; N, 6,97; S, 5,14.

Beispiel 3

Eine gerührte Suspension von 6,7 g (0,016 Mol) Amoxicillin-Trihydrat in 125 ml destilliertem Wasser wird in einer Portion mit 16 ml 1N wässriger Natronlauge versetzt. Das Amoxicillin löst sich rasch auf; nach 2 Minuten gibt man eine Lösung von 4,99 g (0,048 Mol) Tetrahydrothiophen-2-ol in 10 ml absolutem Aethanol hinzu. Man rührt noch während 2 Minuten bei 25°C, friert die erhaltene Lösung rasch ein und lyophilisiert während 19 Stunden. Man erhält 8,36 g /2S-[2α,5α,6β(S*)]/-6-/[Tetrahydro-2-thienylamino-(4-hydroxyphenyl)-acetyl]amino/-3,3-dimethyl-7-oxo-4-thia-1-azabicyclo[3.2.0]heptan-2-carbonsäure-Natriumsalz-Hydrat als hellgelbes Pulver von stechendem Geruch.

Anal. Berechnet für $C_{20}H_{24}N_3NaO_5S_2 \cdot 2H_2O$:
C, 47,14; H, 5,54; N, 8,25; S, 13,20
Gefunden: C, 47,73; H, 5,75; N, 7,70; S, 12,58.

Beispiel 4

Eine gerührte Suspension von 5,028 g (0,012 Mol) Amoxicillin-Trihydrat in 150 ml destilliertem Wasser wird in einer Portion mit 12 ml 1N wässriger Natronlauge versetzt. Das Amoxicillin löst sich rasch auf. Nach 2 Minuten gibt man eine Lösung von 4,83 g (0,036 Mol) 5-Aethyl-3-fluortetrahydro-2-furanol in 6 ml absolutem Aethanol hinzu und rührt während 2 Minuten bei 25°C. Die erhaltene Lösung wird rasch eingefroren und während 19 Stunden lyophilisiert. Man erhält 7,44 g /2S-[2α,5α,6β(S*)]/-6-/bis[Aethyl-3-fluor-2-tetrahydrofuranylamino-(4-hydroxyphenyl)acetyl]-amino/-3,3-dimethyl-7-oxo-4-thia-1-azabicyclo[3.2.0]heptan-2-carbonsäure-Natriumsalz-Hydrat als weisses Pulver.

Anal. Berechnet für $C_{28}H_{36}F_2N_3NaO_7S.2\ H_2O$:
C, 51,29; H, 6,14; N, 6,41; S, 4,89; F, 5,79
Gefunden: C, 50,70; H, 6,25; N, 6,20; S, 5,17; F, 5,39.

Das als Ausgangsmaterial verwendete 5-Aethyl-3-fluor-tetrahydro-2-furanol kann wie folgt hergestellt werden:

Eine aus 27,6 g (1,2 Mol) Natrium und 600 ml absolutem Aethanol hergestellte Lösung wird tropfenweise mit 192 g (1,2 Mol) Diäthylmalonat versetzt. Gegen Ende der Zugabe werden weitere 200 ml Aethanol zur zähen Suspension zugegeben. Die Mischung wird anschliessend noch während 30 Minuten gerührt. Anschliessend versetzt man tropfenweise mit 86,4 g (1,2 Mol) 1,2-Epoxybutan unter Kühlen in einem Eisbad, so dass die Temperatur nicht über 40°C steigt. Das ursprünglich gebildete Salz geht langsam in Lösung; mit zunehmender Fortdauer der Zugabe fällt ein zweites Salz aus. Weitere 200 ml Aethanol werden hinzugefügt, um das Rühren zu erleichtern. Nach Rühren über Nacht versetzt man mit 68 ml Eisessig (pH 6), filtriert den entstandenen Niederschlag ab und wäscht mit Aether. Man entfernt das Lösungsmittel unter vermindertem Druck und löst den Rückstand in 2 Liter Aether. Die ätherische Phase wird über Magnesiumsulfat getrocknet und unter vermindertem Druck eingedampft. Durch Destillation des Rückstandes erhält man 166,5 g (75%) 5-Aethyl-tetrahydro-2-oxo-furan-3-carbonsäureäthylester vom Siedepunkt 112°C (0,2 mmHg).

Eine unter Argon gerührte Suspension von 21,4 g Natriumhydrid in 1 Liter trockenem Toluol wird tropfenweise mit 166 g (0,89 Mol) 5-Aethyl-tetrahydro-2-oxo-furan-3-carbonsäureäthylester versetzt. Nach beendeter Zugabe wurde die erhaltene Mischung während 18 Stunden bei 25°C gerührt. Die erhaltene dicke Suspension wird anschliessend unter Argon mit Perchlorylfluorid versetzt. Die innere Temperatur wird mit einem auf -20° bis -30°C gekühlten Trockeneis/Aceton-Bad zwischen 0-5°C gehalten. Wenn die innere Temperatur langsam die Badtemperatur erreicht, wird während weiteren 15 Minuten Perchlorylfluorid eingeleitet, gefolgt von einem raschen Strom von Argon bei Raumtemperatur. Die Mischung wird anschliessend unter Nachwaschen mit Aether über Cellit filtriert und unter vermindertem Druck eingedampft. Durch Destillation des Rückstandes erhält man 162,5 g (89%) 5-Aethyl-3-fluortetrahydro-2-oxo-furan-3-carbonsäureäthylester vom Siedepunkt 103-108°C (0,3 mmHg).

Anal. Berechnet für $C_9H_{13}FO_4$:
C, 52,94; H, 6,42; F, 9,30
Gefunden: C, 53,00; H, 6,36; F, 9,39.

Eine Lösung von 178 g (3,17 Mol) Kaliumhydroxid in 712 ml Wasser wird mit 162 g (0,79 Mol) 5-Aethyl-3-fluor-tetrahydro-2-oxo-furan-3-carbonsäureäthylester versetzt. Man rührt die Mischung während 18 Stunden bei 25°C, und entfernt anschliessend das Wasser bei 50-55°C im Hochvakuum. Nach Ansäuern des Rückstandes mit 1N Schwefelsäure auf pH 2 extrahiert man gründlich mit Essigester. Die Essigesterlösung wird über Magnesiumsulfat getrocknet und unter vermindertem Druck eingedampft. Man erhält 154 g rohes Malonsäurederivat. Die Decarboxylierung erfolgt durch Erhitzen des unverdünnten Malonsäurederivates während 5 Stunden in einem Oelbad auf 150-155°C. Durch Destillation des Rückstandes erhält man 79 g (75%) 5-Aethyl-3-fluor-dihydro-2(3H)-furanon vom Siedepunkt 72-74°C (0,3 mmHg).

Anal. Berechnet für $C_6H_9FO_2$:
C, 54,54; H, 6,87; F, 14,38
Gefunden: C, 54,71; H, 6,93; F, 14,41.

Eine Lösung von 19,8 g (0,15 Mol) 5-Aethyl-3-fluor-dihydro-2(3H)-furanon in 500 ml trockenem Tetrahydrofuran wird bei -70°C mit 128 ml einer 1,4N Lösung von Dibal-H in Toluol versetzt. Die Lösung wird anschliessend während einer Stunde bei -70°C gerührt und anschliessend vorsichtig auf Eiswasser gegossen. Die erhaltene Mischung wird anschliessend mit 200 ml 1N Salzsäure auf pH 1 eingestellt und mit Methylenchlorid extrahiert. Die Lösung wird über Magnesiumsulfat getrocknet und unter vermindertem Druck eingedampft. Man erhält 20 g Rohprodukt. Durch Destillation erhält man 13,13 g 5-Aethyl-3-fluortetrahydro-2-furanol vom Siedepunkt 45-46°C (0,1 mmHg).

Anal. Berechnet für $C_6H_{11}FO_2$:
C, 53,72; H, 8,27; F, 14,16
Gefunden: C, 53,70; H, 8,33; F, 14,45.

## Beispiel 5

| Bestandteile | in mg/Tablette | | |
|---|---|---|---|
| 1./2S-[2α,5α,6β(S*)]/-6-/[Tetrahydro-2-thienyl-amino-(4-hydroxyphenyl)-acetyl]amino/-3,3-dimethyl-7-oxo-4-thio-1-azabicyclo[3.2.0]heptan-2-carbonsäure-Natriumsalz-Hydrat | 100 | 250 | 500 |
| 2.Lactose | 147,5 | 100 | 97,5 |
| 3.Vorgelatinisierte Stärke | 25 | 30 | 60 |
| 4.Modifizierte Stärke | 25 | 50 | 60 |
| 5.Kornstärke | 25 | 50 | 60 |
| 6.Magnesiumstearat | 2,5 | 5 | 7,5 |
| Tablettengewicht | 325 mg | 500 mg | 785 mg |

Verfahren:

Die Bestandteile 1-5 werden in einem geeigneten Mischer vermischt, mit Wasser granuliert und über Nacht in einem geeigneten Ofen getrocknet. Nach Malen vermischt man mit Bestandteil 6 und verpresst auf einer geeigneten Presse.

Beispiel 6

In Analogie zu Beispiel 5 können Tabletten hergestellt werden, welche /2S-[2α,5α,6β(S*)]/-6-[Bis(tetrahydro-2H-pyran-2-yl)amino]-/[(hydroxyphenyl)acetyl]amino/-3,3-dimethyl-7-oxo-4-thia-1-azabicyclo[3.2.0]heptan-2-carbonsäure-Natriumsalz-Hydrat als Wirkstoff enthalten.

Beispiel 7

In Analogie zu Beispiel 5 können Tabletten hergestellt werden, welche 2S-[2α,5α,6β(S*)-6-/Bis[Aethyl-3-fluor-2-tetrahydrofuranylamino-(4-hydroxyphenyl)acetyl]amino/-3,3-dimethyl-7-oxo-4-thia-1-azabicyclo[3.2.0]heptan-2-carbonsäure-Natriumsalz als Wirkstoff enthalten.

Beispiel 8

Herstellung von Kapseln

| Bestandteile | in mg/Kapsel | | |
|---|---|---|---|
| 1. /2S-[2α,5α,6β(S*)]/-6-[Bis-(tetrahydro-2H-pyran-2-yl)-amino]-/[(4-hydroxyphenyl)-acetyl]amino/-3,3-dimethyl-7-oxo-4-thia-1-azabicyclo-[3.2.0]heptan-2-carbonsäure-Natriumsalz-Hydrat | 100 | 250 | 500 |
| 2. Lactose | 99 | 148 | --- |
| 3. Kornstärke | 20 | 30 | 57 |
| 4. Talk | 5 | 10 | 15 |
| 5. Magnesiumstearat | 1 | 2 | 3 |
| Kapselfüllgewicht | 225 mg | 440 mg | 575 mg |

Verfahren:

Die Bestandteile 1-3 werden in einem geeigneten Mixer vermischt. Nach Vermahlen wird die Mischung mit den Bestandteilen 4 und 5 vermischt und auf einer geeigneten Abfüllmaschine in Kapseln abgefüllt.

Beispiel 9

In Analogie zu Beispiel 8 können Kapseln hergestellt werden, welche /2S-[2α,5α,6β(S*)]/-6-[Tetrahydro-2-thienyl-amino-(4-hydroxyphenyl)acetyl]amino/-3,3-dimethyl-7-oxo-4-thia-1-azabicyclo[3.2.0]heptan-2-carbonsäure-Natriumsalz-Hydrat als Wirkstoff enthalten.

Beispiel 10

In Analogie zu den Angaben in Beispiel 8 können Kapseln hergestellt werden, welche /2S-[2α,5α,6β(S*)]/-6-/Bis[aethyl-3-fluor-2-tetrahydrofuranylamino-(4-hydroxyphenyl)acetyl]amino/-3,3-dimethyl-7-oxo-4-thia-1-azabicyclo[3.2.0]heptan-2-carbonsäure-Natriumsalz-Hydrat als Wirkstoff enthalten.

Beispiel 11

Formulierung

| | 100 mg/ Behälter | 200 mg/ Behälter | 500 mg/ Behälter | 1 Liter |
|---|---|---|---|---|
| /2S-[2α,5α,6β(S*)]/-6-[Bis-(tetrahydro-2H-pyran-2-yl)-amino]-/[(4-hydroxyphenyl)-acetyl]amino/-3,3-dimethyl-7-oxo-4-thia-1-azabicyclo-[3.2.0]heptan-2-carbonsäure-Natriumsalz | 110 | 220 | 550 | 200.0 g |
| Wasser für die Injektion | 0,55 ml | 1,10 ml | 2,75ml | 1000 ml |

Herstellungsverfahren

200 g eines lyophilisierten Pulvers von /2S-[2α,5α,6β(S*)]/-6-[Bis(tetrahydro-2H-pyran-2-yl)amino]-/[(4-hydroxyphenyl)acetyl]amino/-3,3-dimethyl-7-oxo-4-thia-1-azabicyclo[3.2.0]heptan-2-carbonsäure-Natriumsalz-Hydrat werden in 800 ml Wasser für die Injektion gelöst. Man

füllt mit Wasser auf 1000 ml auf. Die Lösung wird anschliessend durch einen sterilen Milliporfilterapparat (0,22 mikron Porositätsfilter) gelassen. Die Lösung kann auch durch einen sterilen Keramikwachsfilter filtriert werden. Das Filtrat wird in einem sterilen Behälter aufgefangen.

Die folgenden Lösungsvolumina werden in geeignete sterile Behälter abgefüllt, um die gewünschte Wirkstoffmenge zu erhalten.

| Menge an gewünschtem Wirkstoff pro Behälter | In Behälter abzufüllendes Volumen |
|---|---|
| 100 mg | 0,55 ml |
| 200 mg | 1,10 ml |
| 500 mg | 2,75 ml |

Die gefüllten Behälter werden in einen geeigneten sterilen Lyophilisator gegeben. Geeignete Gefässe werden mit sterilen Thermoelementen versehen, um die Temperatur der jeweiligen Inhalte während des Lyophilisationszyklus zu kontrollieren. Die Lösungen in den Behältern werden auf eine Temperatur von -40°C eingefroren. Man evakuiert und erhöht die Temperatur der Lyophilisatorfächer nach und nach auf 35°C bis 40°C. Das Vakuum wird durch Einblasen von sterilem Stickstoff aufgefüllt. Die Behälter werden mit sterilen Gummistopfen verschlossen. Ein dreiteiliger metalliger Flaschenverschluss wird jeweils über den Stopfen und den Abschluss des Behälters gestülpt.

Für die Wiederaufbereitung des lyophilisierten Pulvers wird steriles Wasser für die Injektion oder Natriumchloridlösung für die Injektion verwendet.

| Behältergrösse | minimale hinzuzufügende Menge an Verdünnungsmittel |
|---|---|
| 100 mg | 0,5 ml |
| 200 mg | 1,0 ml |
| 500 mg | 2,5 ml |

Beispiel 12

In Analogie zu den Angaben in Beispiel 11 wird eine sterile Injektionslösung hergestellt, welche /2S-[2α,5α,6β(S*)]/-6-/[Tetrahydro-2-thienylamino-(4-hydroxyphenyl)acetyl]amino/-3,3-dimethyl-7-oxo-4-thia-1-azabicyclo[3.2.0]heptan-2-carbonsäure-Natriumsalz-Hydrat als Wirkstoff enthält.

## Patentansprüche

1. Acylderivate der Formel:

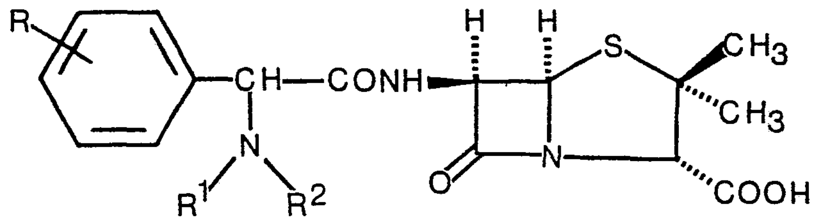

II

worin R Wasserstoff oder Hydroxy und $R^1$ und $R^2$ unabhängig voneinander Wasserstoff oder einen unsubstituierten oder einfach oder mehrfach durch niederes Alkyl und/oder Halogen substituierten, gesättigten, ein Heteroatom enthaltenden, heterocyclischen 5- oder 6-gliedrigen Ring bedeuten, der als Heteroatom ein Sauerstoff- oder Schwefelatom enthält, wobei der besagte Ring über das dem besagten Heteroatom benachbarte Kohlenstoffatom mit dem Stickstoffatom verknüpft ist, mit der Massgabe, dass mindestens einer der Reste $R^1$ und $R^2$ nicht Wasserstoff bedeutet, sowie leicht hydrolysierbare Ester und Salze davon und Hydrate von Verbindungen der Formel II und ihren Estern und Salzen.

2. /2S-[2α,5α,6β(S*)]/-6-/[2-Tetrahydrofuranylamino-(4-hydroxyphenyl)acetyl]amino/-3,3-dimethyl-7-oxo-4-thia-1-azabicyclo[3.2.0]heptan-2-carbonsäure, sowie Salze dieser Verbindung und Hydrate dieser Verbindung und seiner Salze.

3. /2S-[2α,5α,6β(S*)]/-6-[bis(Tetrahydro-2H-pyran-2-yl)amino]-/[(4-hydroxyphenyl)acetyl]amino/-3,3-dimethyl-7-oxo-4-thia-1-azabicyclo[3.2.0]heptan-3-carbonsäure, sowie Salze dieser Verbindung und Hydrate dieser Verbindung und

seiner Salze.

4. /2S-[2α,5α,6β(S*)]/-6-/[Tetrahydro-2-thienylamino-(4-hydroxyphenyl)acetyl]amino/-3,3-dimethyl-7-oxo-4-thia-1-azabicyclo[3.2.0]heptan-2-carbonsäure, sowie Salze dieser Verbindung und Hydrate dieser Verbindung und seiner Salze.

5. /2S-[2α,5α,6β(S*)]/-6-/bis[5-Aethyl-3-fluor-2-tetrahydrofuranylamino-(4-hydroxyphenyl)acetyl]amino/-3,3-dimethyl-7-oxo-4-thia-1-azabicyclo[3.2.0]heptan-2-carbonsäure, sowie Salze dieser Verbindung und Hydrate dieser Verbindung und seiner Salze.

6. Eine Verbindung der allgemeinen Formel

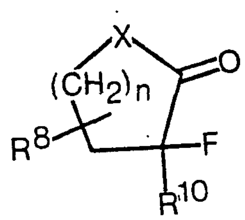

worin X Sauerstoff oder Schwefel, $R^8$ niederes Alkyl, n die Zahl 1 oder 2 und $R^{10}$ Wasserstoff oder niederes Alkoxycarbonyl bedeuten.

7. Eine Verbindung der allgemeinen Formel

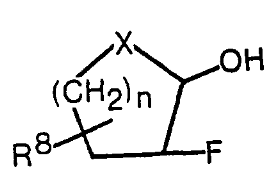

worin X Sauerstoff oder Schwefel, $R^8$ niederes Alkyl und n die Zahl 1 oder 2 bedeuten.

8. Acylderivate nach einem der Ansprüche 1-5 als pharmazeutische Wirkstoffe.

9. Acylderivate nach einem der Ansprüche 1-5 als pharmazeutische Wirkstoffe zur Bekämpfung bzw. Verhütung von Infektionen.

10. Pharmazeutische Präparate, enthaltend ein Acylderivat gemäss einem der Ansprüche 1-5 als Wirkstoff.

11. Pharmazeutisches Präparat zur Bekämpfung bzw. Verhütung von Infektionen, enthaltend ein Acylderivat gemäss einem der Ansprüche 1-5 als Wirkstoff.

12. Pharmazeutische Präparate gemäss Anspruch 10 oder 11, dadurch gekennzeichnet, dass diese Präparate wässrige Formulierungen darstellen.

13. Verfahren zur Herstellung der Acylderivate gemäss einem der Ansprüche 1-5, dadurch gekennzeichnet, dass man

a) zur Herstellung einer Verbindung der allgemeinen Formel II, ein Alkalimetallsalz einer Verbindung der allgemeinen Formel

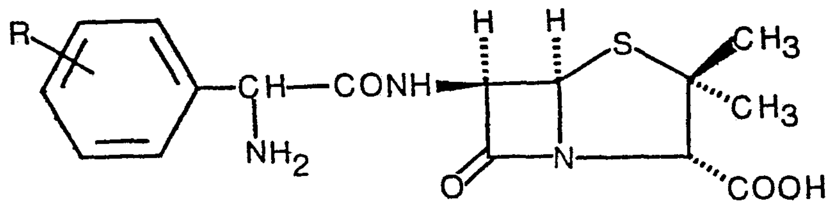

I

worin R Wasserstoff oder Hydroxy bedeutet, mit einer Verbindung der Formel

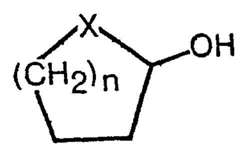

III

worin X Sauerstoff oder Schwefel und n die Zahl 1 oder 2 bedeuten, und die in einer oder in mehreren Stellungen durch niederes Alkyl und/oder Halogen substituiert sein kann, umsetzt,

oder

b) zur Herstellung eines leicht hydrolysierbaren Esters einer Verbindung der allgemeinen Formel II, eine Carbonsäure der allgemeinen Formel II entsprechend verestert,

oder

c) zur Herstellung von Salzen oder Hydraten von Verbindungen der allgemeinen Formel II, oder zur Herstellung von Hydraten dieser Salze, eine Verbindung der allgemeinen Formel II in ein Salz oder in ein Hydrat oder in ein Hydrat eines solchen Salzes überführt.

14. Verwendung von Acylderivaten gemäss einem der Ansprüche 1-5 bei der Bekämpfung bzw. Verhütung von Krankheiten.

15. Verwendung von Acylderivaten gemäss einem der Ansprüche 1-5 bei der Bekämpfung bzw. Verhütung von Infektionen.

***

Patentansprüche

"für OESTERREICH"

1. Verfahren zur Herstellung von Acylderivaten der Formel:

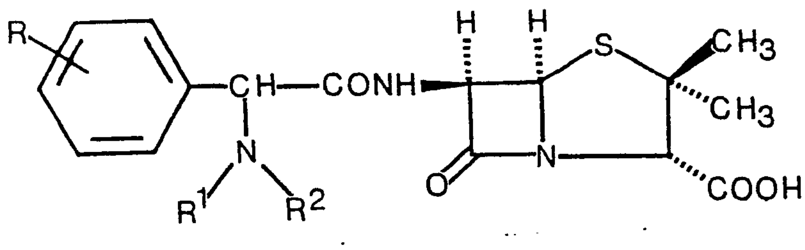

II

worin R Wasserstoff oder Hydroxy und $R^1$ und $R^2$ unabhängig voneinander Wasserstoff oder einen unsubstituierten oder einfach oder mehrfach durch niederes Alkyl und/oder Halogen substituierten, gesättigten, ein Heteroatom enthaltenden, heterocyclischen 5- oder 6-gliedrigen Ring bedeuten, der als Heteroatom ein Sauerstoff- oder Schwefelatom enthält, wobei der besagte Ring über das dem besagten Heteroatom benachbarte Kohlenstoffatom mit dem Stickstoffatom verknüpft ist, mit der Massgabe, dass mindestens einer der Reste $R^1$ und $R^2$ nicht Wasserstoff bedeutet,

sowie von leicht hydrolysierbaren Estern und Salzen davon und von Hydraten von Verbindungen der Formel II und ihren Estern und Salzen, dadurch gekennzeichnet, dass man

a) zur Herstellung einer Verbindung der allgemeinen Formel II, ein Alkalimetallsalz einer Verbindung der allgemeinen Formel

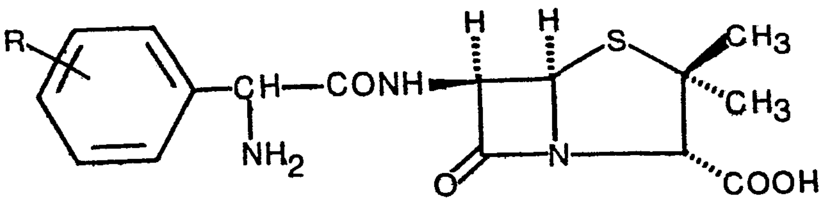

I

worin R Wasserstoff oder Hydroxy bedeutet, mit einer Verbindung der Formel

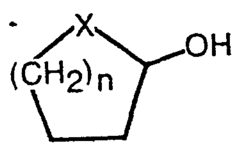

III

worin X Sauerstoff oder Schwefel und n die Zahl 1 oder 2 bedeuten, und die in einer oder in mehreren Stellungen durch niederes Alkyl und/oder Halogen substituiert sein kann, umsetzt,

oder

b) zur Herstellung eines leicht hydrolysierbaren Esters einer Verbindung der allgemeinen Formel II, eine Carbonsäure der allgemeinen Formel II entsprechend verestert,

oder

c) zur Herstellung von Salzen oder Hydraten von Verbindungen der allgemeinen Formel II, oder zur Herstellung von Hydraten dieser Salze, eine Verbindung der allgemeinen Formel II in ein Salz oder in ein Hydrat oder in ein Hydrat eines solchen Salzes überführt.

2. Verfahren gemäss Anspruch 1, dadurch gekennzeichnet, dass man /2S-[2α,5α,6β(S*)]/-6-/[2-Tetrahydrofuranylamino-(4-hydroxyphenyl)acetyl]amino/-3,3-dimethyl-7-oxo-4-thia-1-azabicyclo[3.2.0]heptan-2-carbonsäure, sowie Salze dieser Verbindung und Hydrate dieser Verbindung und seiner Salze herstellt.

3. Verfahren gemäss Anspruch 1, dadurch gekennzeichnet, dass man /2S-[2α,5α,6β(S*)]/-6-[bis(Tetrahydro-2H-pyran-2-yl)amino]-/[(4-hydroxyphenyl)acetyl]amino/-3,3-dimethyl-7-oxo-4-thia-1-azabicyclo[3.2.0]heptan-2-carbonsäure, sowie Salze dieser Verbindung und Hydrate dieser Verbindung und seiner Salze herstellt.

4. Verfahren gemäss Anspruch 1, dadurch gekennzeichnet, dass man /2S-[2α,5α,6β(S*)]/-6-/[Tetrahydro-2-thienylamino-(4-hydroxyphenyl)acetyl]amino/-3,3-dimethyl-7-oxo-4-thia-1-azabicyclo[3.2.0]heptan-2-carbonsäure, sowie Salze dieser Verbindung und Hydrate dieser Verbindung und seiner Salze herstellt.

5. Verfahren gemäss Anspruch 1, dadurch gekennzeichnet, dass man /2S-[2α,5α,6β(S*)]/-6-/bis[5-Aethyl-3-fluor-2-tetrahydrofuranylamino-(4-hydroxyphenyl)acetyl]amino/-3,3-dimethyl-7-oxo-4-thia-1-azabicyclo[3.2.0]heptan-2-carbonsäure, sowie Salze dieser Verbindung und Hydrate dieser Verbindung und seiner Salze herstellt.